(19) Europäisches Patentamt

European Patent Office

Office européen des brevets

(11) **EP 4 717 248 A2**

(12) **EUROPEAN PATENT APPLICATION**

(43) Date of publication:
**01.04.2026 Bulletin 2026/14**

(21) Application number: **25203225.5**

(22) Date of filing: **19.09.2025**

(51) International Patent Classification (IPC):
*A61K 6/15* (2020.01)        *A61K 6/818* (2020.01)
*A61K 6/836* (2020.01)       *A61K 6/887* (2020.01)
*A61K 6/71* (2020.01)        *A61K 6/76* (2020.01)
*A61K 6/61* (2020.01)        *A61K 6/77* (2020.01)

(52) Cooperative Patent Classification (CPC):
(C-Sets available)
**A61K 6/15; A61K 6/71; A61K 6/76; A61K 6/887;**
A61K 6/61; A61K 6/77                       (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **28.09.2024 JP 2024169694**

(71) Applicant: **Shofu Inc.**
**Kyoto-shi, Kyoto 605-0983 (JP)**

(72) Inventors:
• **TAKAHASHI, Shuhei**
  **Kyoto-shi, Kyoto, 605-0983 (JP)**
• **OBATA, Atsushi**
  **Kyoto-shi, Kyoto, 605-0983 (JP)**

(74) Representative: **Eisenführ Speiser**
**Patentanwälte Rechtsanwälte PartGmbB**
**Gollierstraße 4**
**80339 München (DE)**

(54) **MANUFACTURING METHOD OF RESIN BLANK FOR DENTAL CUTTING AND MACHINING**

(57)    To provide a dental resin blank for cutting and machining which has excellent versatility and productivity and is imparted with high mechanical strength.

To provide a dental resin blank for cutting and machining by the manufacturing method of a dental resin blank for cutting and machining, comprising step (1) of contacting at least one type of porous inorganic filler with a polymerizable monomer, step (2) of impregnating the porous inorganic filler with the polymerizable monomer to obtain a granular powder, and step (3) of placing the granular powder into a mold and applying pressure and heat to cure.

EP 4 717 248 A2

**(Cont. next page)**

(52) Cooperative Patent Classification (CPC): (Cont.)

C-Sets
**A61K 6/887, C08L 33/10**

**Description**

BACKGROUND OF THE INVENTION

Field of the Invention

The present invention relates to a manufacturing method of a dental resin blank for cutting and machining.

Description of the Related Art

In recent years, techniques to prepare a prosthesis device using the dental CAD/CAM system has been spread rapidly. Generally, in such systems, a mill blank (blank) in the form of a disc or a block has been mounted on a dental milling machine to perform cutting and machining.

As this blank, there has been various materials including metallic materials such as titanium alloy, ceramic materials such as zirconia, alumina, and lithium disilicate, and composite resin materials made from polymerizable monomers and inorganic fillers. It is possible to provide various prosthesis devices by selecting the blank material according to the purpose.

Among these, a dental resin blank for cutting and machining, made from a composite resin material, has moderate hardness that avoids damaging an opposing tooth and excellent impact resistance, and therefore has been processed into a dental prosthesis device and used clinically.

For example, Japanese Unexamined Patent Application Publication No. H10-323353 discloses a manufacturing method of a dental resin blank for cutting and machining, wherein a polymerizable monomer and an inorganic filler are kneaded to prepare a paste-like product and then polymerized and cured within a mold of the desired shape. This method has an advantage of easily manufacturing a dental resin blank for cutting and machining which contains 20 to 70 % by weight of an inorganic filler. However, because the dental resin blank is manufactured via the paste-like product, there is a limitation on the filling amount of the inorganic filler, and therefore it is difficult to impart sufficient mechanical property.

International Publication No. 2014/021343 discloses a manufacturing method of a dental resin blank for cutting and machining in which after the inorganic filler is press-molded in advance, the molded body is immersed with a polymerizable monomer and then polymerized by heating. This manufacturing method allows for high filling of inorganic filler nanoparticles and can provide a dental resin blank for cutting and machining having excellent polishing property. However, since it is necessary to impregnate the molded body with a polymerizable monomer, there is a limitation on the viscosity and the component of the polymerizable monomer, and therefore it is difficult to impart sufficient mechanical property.

Japanese Unexamined Patent Application Publication No. 2023-035918 discloses a manufacturing method of a dental resin blank for cutting and machining, which comprises dispersing a polymerizable monomer and an inorganic filler, stirring under vacuum to prepare a granular powder, and then compress-molding the prepared powder. It is described that this manufacturing method can provide a dental resin blank for cutting and machining having excellent gradation by layering and molding granular powders having different color tones. However, in this method, because it is difficult to prepare uniform granular powder, the dental resin blank for cutting and machining has density variations. Therefore, there is a problem in that appearance of the blank is poor, cracks and fractures are easily occurred and it is impossible to impart sufficient mechanical strength.

SUMMARY OF THE INVENTION

Technical Problem

As described above, the prior art discloses several manufacturing methods of a dental resin blank for cutting. However, there has been a limitation on the usable polymerizable monomers in these manufacturing methods of a resin blank, therefore it has been difficult to impart sufficient mechanical strength and there has been a problem in manufacturing stability.

Therefore, an object of the present invention is to provide a dental resin blank for cutting and machining which has excellent versatility and productivity and is imparted with high mechanical strength.

Solution to Problem

The present inventors have made studies on a manufacturing method of a dental resin blank for cutting and machining, which is excellent in productivity, is easily product regardless of the composition or components of the polymerizable monomer and can impart sufficient mechanical strength. As a result, it has been found that the above described problem can be improved by molding granular powder in which polymerizable monomer is impregnated into at least one type of porous inorganic filler and by polymerization and curing. Details of the present invention are described below.

That is, the present invention provides a manufacturing method of a dental resin blank for cutting and machining, comprising step (1) of contacting at least one type of porous inorganic filler with a polymerizable monomer, step (2) of impregnating the porous inorganic filler with the polymerizable monomer to obtain a granular powder, and step (3) of placing the granular powder into a mold and applying pressure and heat to cure.

In the present invention, a specific surface area of the porous inorganic filler may be 10 to 300 m$^2$/g.

In the present invention, a pore volume of the porous inorganic filler may be 0.01 to 0.20 cc/g.

In the present invention, the step (2) of impregnating the porous inorganic filler with the polymerizable monomer to obtain a granular powder may be performed under vacuum condition.

Advantageous Effects of Invention

In the present invention, after contacting at least one type of porous inorganic filler with a polymerizable monomer, a granular powder is prepared by impregnating the porous inorganic filler with the polymerizable monomer, the granular powder is placed into a mold, and a part or all of the polymerizable monomer encapsulated within the granular powder is discharged during pressurization and heating to cure. Therefore, the present invention can provide a dental resin blank for cutting and machining which has excellent versatility and productivity and is imparted with high mechanical strength.

DETAILED DESCRIPTION OF THE PREFERRED EMBODIMENTS

The details of the present invention are described below.

The manufacturing method of a dental resin blank for cutting and machining of the present invention comprises step (1) of contacting at least one type of porous inorganic filler with a polymerizable monomer, step (2) of impregnating the porous inorganic filler with the polymerizable monomer to obtain a granular powder, and step (3) of placing the granular powder into a mold and applying pressure and heat to cure.

The porous inorganic filler used in the dental cutting resin blank of the present invention refers to an inorganic filler having at least one pore. The presence or absence of pores in an inorganic filler can be measured, for example, by gas adsorption or mercury intrusion porosimetry. More specifically, the porous inorganic filler in the present invention refers to one having a pore volume of 0.001 cc/g or more as measured by gas adsorption.

The shape of the porous inorganic filler in the present invention is not particularly limited, and it is possible to use a spherical shape and an irregularly shape (for example, needle-like, plate-like, ground-like and scaly-shape). In particular, by forming the porous inorganic filler into a spherical shape, it is possible to impart a high specific surface area and a high pore volume, and therefore it is possible to easily impregnate with polymerizable monomers. Furthermore, by using spherical porous inorganic filler material, it is possible to impart excellent surface smoothness to the dental resin blank for cutting and machining, and to additionally impart high mechanical strength.

The shape of the porous inorganic filler can be defined by its circularity. In the present invention, a circularity of 0.8 or greater refers as spherical, and a circularity of less than 0.8 refers as amorphous.

The circularity can be determined based on the projected area and the perimeter of particle obtained by observing the inorganic filler material using a scanning electron microscope (SEM) and processing the observed image with an image analysis device. In the case that S is defined as the projected area of the inorganic filler obtained by image processing and L is defined as the perimeter of the particle, the circularity is calculated by following formula 1.

$$[\text{Formula 1}] \ \text{Circularity} = (4 \pi S) / (L^2)$$

A reproducible, substantially constant value is obtained by averaging the values from 100 or more samples.

The 50% particle diameter of the porous inorganic filler in the present invention may be 0.01 to 50 $\mu$m, and may be 0.1 to 10 $\mu$m. The 50% particle diameter may be measured using a general laser diffraction particle size analyzer. When the 50% particle diameter exceeds 50 $\mu$m, there is a case where the mechanical strength of the obtained dental resin blank for cutting and machining decreases and surface smoothness decreases. When the 50% particle diameter is less than 0.01 $\mu$m, there is a case where the specific surface area and pore volume of the porous inorganic filler becomes excessive to reduce the inorganic filler content in the dental resin blank for cutting and machining to decrease mechanical strength.

The specific surface area of the porous inorganic filler in the present invention may be 10 to 300 m$^2$/g, and may be 20 to 250 m$^2$/g. Furthermore, the pore volume of the porous inorganic filler may be 0.01 to 0.20 cc/g, and may be 0.05 to 0.15 cc/g. The specific surface area and the pore volume can be measured using a conventional nitrogen adsorption method. By using a porous inorganic filler having these characteristics, a polymerizable monomer easily penetrates into the pore interior, therefore it is possible to easily manufacture a granular particle.

When the specific surface area and/or the pore volume is too small, there is a case where it is difficult to manufacture uniform granular particle. When the specific surface area and/or the pore volume is too large, there is a case where an excessive amount of polymerizable monomer with respect to the inorganic filler is required, the inorganic filler content in the dental resin blank for cutting and machining decreases, which leads to decrease of mechanical strength.

As the component of the porous inorganic filler in the present invention, any known components generally used in the field of dental materials can be used without any limitations. Among these, it is possible to contain silicon dioxide and an oxide containing at least one metal element. Specifically, the porous inorganic filler in the present invention can substantially consist of silicon dioxide and an oxide containing at least one metal element, or can consist of silicon dioxide and an oxide containing at least one metal element. In the present invention, the porous inorganic filler substantially consisting of silicon dioxide and an oxide containing at least one metal element means to intend to allow the presence of unavoidable impurities in addition to silicon dioxide and an oxide containing at least one metal element. As the at least one metal element, metal elements such as Al, Ba, Bi, Ca, Ce, Co, Cu, Er, Fe, Hf, Ho, In, La, Mg, Mn, Nd, Ni, Pb, Sb, Sn, Sr, Ta, Ti, Y, Yb, Zn and Zr can be used without any limitation. Specific examples of metal oxides include oxides of Al, Ba, Ca, Co, Cu, Fe, Hf, La, Mg, Ni, Sr, Ti, Zn and Zr. Furthermore, by selecting an oxide of a metal element with a relatively high atomic number as the metal element, it is possible to impart X-ray contrast to the dental resin blank for cutting and machining, and it is also possible to more easily match the refractive index with that of the polymerizable monomer.

The content of the oxide containing at least one metal element contained in the porous inorganic filler may be 1 to 80 % by weight in terms of oxide, and more preferably 3 to 50 % by weight. When the content of the oxide containing at least one metal element contained in the porous inorganic filler is less than 1 % by weight or exceeds 80 % by weight, there is a case where it becomes difficult to adjust the refractive index to the polymerizable monomer.

In the present invention, commonly used known inorganic filler other than the porous inorganic filler may be used in combination with the porous inorganic filler as other inorganic filler. By compounding other inorganic filler, it is possible to increase the inorganic filler content in the dental resin blank for cutting and machining to improve mechanical strength.

Specific examples of the other inorganic filler in the present invention include silica, aluminum silicate, alumina, titania, zirconia, various glasses (including fluoride glass, borosilicate glass, soda glass, barium glass, barium aluminum silica glass, strontium glass, glass containing zirconium, glass ceramics, fluoroaluminosilicate glass and synthetic glass by a sol-gel method), Aerosil (registered trademark), calcium fluoride, strontium fluoride, calcium carbonate, kaolin, clay, mica, aluminum sulfate, calcium sulfate, barium sulfate, titanium oxide, calcium phosphate, hydroxyapatite, calcium hydroxide, strontium hydroxide and zeolite.

As such other inorganic filler, a filler having any shape including spherical or amorphous (e.g., a needle shape, a plate shape, a crushed shape or a scale shape) may be used. In particular, in the case of an amorphous inorganic filler, that is, a filler having a needle shape, a plate shape, a crushed shape or a scale shape, it is possible to impart an effect that suppresses crack propagation and to improve the mechanical strength of the dental resin blank for cutting and machining. The 50% particle diameter of the other inorganic filler is selected appropriately according to the purpose and may be, for example, 0.01 to 100 $\mu$m or 0.1 to 10 $\mu$m. When the 50% particle diameter of the other inorganic filler is less than 0.01 $\mu$m, there is a case where the specific surface area become excessive to decrease the inorganic filler amount. Furthermore, when the particle diameter exceeds 100 $\mu$m, there is a case where it becomes a point of fracture initiation to decrease mechanical strength.

The inorganic filler content which is a total of the contents of the porous inorganic filler and other inorganic filler in the dental resin blank for cutting and machining of the present invention may be set without particular limitation. Considering physical properties, the inorganic filler content in the dental resin blank for cutting and machining may be 20 to 99 % by weight, and may be 40 to 90 % by weight. When the inorganic filler content in the dental resin blank for cutting and machining is less than 20 % by weight, there is a case where sufficient mechanical property or hardness is not imparted. Furthermore, when the inorganic filler content exceeds 99 % by weight, there is a case where it causes brittleness and sufficient mechanical property is not imparted.

The ratio of the porous inorganic filler to the other inorganic filler in the dental resin blank for cutting and machining of the present invention is preferably 100:0 to 1:99, and more preferably 90:10 to 10:90 by weight. When the ratio of the porous inorganic filler with respect to the other inorganic filler is too low, there is a possibility that it becomes difficult to manufacture a granular particle.

It is preferable that the inorganic filler (the porous inorganic filler and other inorganic filler) in the present invention is surface-treated in order to improve compatibility with the polymerizable monomer. The method of surface treatment is not particularly limited, and it is possible to select from common treatments such as silane treatment or plasma treatment according to the purpose. Among these, surface treatments using treatment materials such as silane coupling material, titanate coupling material or aluminate coupling material is preferable. Examples of the silane coupling material include $\gamma$-methacryloxypropyl trimethoxysilane and $\gamma$-methacryloxypropyl triethoxysilane. Preferably, $\gamma$-methacryloxypropyl tri-methoxysilane is used.

The polymerizable monomer in the present invention is not particularly limited, and for example, known polymerizable monomer generally used in the field of dental material can be used. As the polymerizable monomer, examples include a general radical polymerizable monomer, and a polymerizable monomer having an acryloyl group and/or a methacryloyl group are preferably usable. Specific examples are as follows.

Examples of a monofunctional polymerizable monomer include (meth)acrylic acid esters such as methyl (meth) acrylate, ethyl (meth)acrylate, butyl (meth)acrylate, hexyl (meth)acrylate, tetrahydrofurfuryl (meth)acrylate, 2-hydroxyethyl (meth)acrylate, grycidyl (meth)acrylate, lauryl (meth)acrylate, cyclohexyl (meth)acrylate, benzil (meth)acrylate, allyl (meth)acrylate, 2-ethoxyethyl (meth)acrylate, methoxy polyethylene glycol (meth)acrylate, glycerol (meth)acrylate and isobornyl (meth)acrylate; silane compounds such as $\gamma$-(meth)acryloyloxy propyltrimethoxysilane and $\gamma$-(meth) acryloyloxy propyltriethoxysilane; nitrogen-containing compounds such as 2-(N,N-dimethylamino) ethyl (meth)acrylate, N-methylol (meth)acrylamide and diacetone (meth)acrylamide.

The resin material for dental milling and machining of the present invention include an inorganic filler in a content of 40 % by weight or more. The resin material for dental milling and machining of the present invention can be obtained, for example, by polymerizing and curing in a die having a desired shape, a paste-like substance prepared by mixing, as the main components, a polymerizable monomer, an inorganic filler, a polymerization catalyst and a colorant. Hereinafter, described are such components capable of being used in the resin material for dental milling and machining of the present invention. For the polymerizable monomer as one of the constituent components, heretofore known polymerizable monomers generally used in dental materials can be used without being particularly limited. Examples of the polymerizable monomer include generally radical polymerizable monomers. Examples of the monofunctional polymerizable monomer include: (meth)acrylic acid esters such as methyl (meth)acrylate, ethyl (meth)acrylate, n-butyl (meth)acrylate, isobutyl (meth)acrylate, tert-butyl (meth)acrylate, pentyl (meth)acrylate, isopentyl (meth)acrylate, neopentyl (meth) acrylate, glycidyl (meth)acrylate, tetrahydrofurfuryl (meth)acrylate, 2-hydroxyethyl (meth)acrylate, 2-hydroxypropyl (meth)acrylate, 3-chloro-2-hydroxypropyl (meth)acrylate, ethylene glycol acetoacetate (meth)acrylate, ethylene glycol mono(meth)acrylate, diethylene glycol mono(meth)acrylate, triethylene glycol mono(meth)acrylate, polyethylene glycol mono(meth)acrylate, methoxydiethylene glycol mono(meth)acrylate, methoxytetraethylene glycol (meth)acrylate, methoxypolyethylene glycol (meth)acrylate, $\beta$-(meth)acryloxyethyl hydrogen phthalate, $\beta$-(meth)acryloxyethyl hydrogen succinate, nonylphenoxyethyl (meth)acrylate, phenoxyethyl (meth)acrylate, phenoxydiethylene (meth)acrylate, N-(2-hydroxy-3-(meth)acryloyloxypropyl)-N-phenylglycine, N-(meth)acryloyl glycine and 4-(meth)acryloyloxyethyl trimellitic acid anhydride; vinyl esters such as vinyl acetate and vinyl propionate; vinyl ethers such as methyl vinyl ether, ethyl vinyl ether, isobutyl vinyl ether and (meth)acrylaldehyde ethyl acetal; alkenylbenzenes such as styrene, vinyltoluene, $\square$-methylstyrene and chlorostyrene; vinyl cyanides such as acrylonitrile and (meth)acrylonitrile; (meth)acrylic acid aldehydes such as (meth)acrylaldehyde and 3-cyano(meth)acrylaldehyde; (meth)acrylic acid amides such as (meth)acrylamide, N-succin(meth)acrylamide and N,N-dimethylmethacrylamide; polymerizable monomers containing an unsaturated carboxylic acid group such as (meth)acrylic acid, vinylacetic acid and crotonic acid, or the metal salts of these; polymerizable monomers containing a phosphoric acid ester group such as acid phosphoethyl (meth)acrylate, acid phosphopropyl (meth)acrylate and 2-(meth)acryloyloxyethylphenyl phosphate, or the metal salts of these; polymerizable monomers containing a sulfonic acid group such as allylsulfonic acid, (meth)acrylsulfonic acid, styrenesulfonic acid and tert-butyl(meth)acrylamide sulfonic acid, or the metal salts of these. A specific example of the radical polymerizable monomer compounds may be bifunctional polymerizable monomers including di(meth)acrylate such as ethylenediol, propylenediol, propanediol, butanediol, hexanediol, octanediol, nonanediol, decanediol, eicosanediol; ethylene glycol di(meth)acrylate, diethylene glycol di(meth)acrylate, triethylene glycol di(meth)acrylate, propylene glycol di(meth)acrylate, tetraethylene glycol di(meth)acrylate, polyethylene glycol di(meth)acrylate, 1,3-butylene glycol di(meth)acrylate, neopentyl glycol di(meth)acrylate; urethane-based polymerizable monomer which is derived from an adduct of a vinyl monomer having a hydroxy group such as 2-hydroxyethyl (meth)acrylate, 2-hydroxypropyl (meth)acrylate and 3-chloro-2-hydroxypropyl (meth)acrylate, and a diisocyanate compound such as hexamethylene diisocyanate, trimethyl hexamethylene diisocyanate, diisocyanate methylcyclohexane, isofluoro diisocyanate and methyl bis(4-cyclohexyl isocyanate); aromatic ring and urethane bond-containing (meth)acrylate-based polymerizable monomer which is derived from an adduct of a vinyl monomer having a hydroxy group such as 2-hydroxyethyl (meth)acrylate, 2-hydroxypropyl (meth)acrylate and 3-chloro-2-hydroxypropyl (meth)acrylate, and an aromatic group-containing diisocyanate compound such as diisocyanate methyl benzene and 4,4'-diphenyl methane diisocyanate; a reactant of 1:2 ratio of (meth)acrylate-based polymerizable monomer which has an aromatic ring and an ether linkage, bisphenol A or hydrogenated bisphenol A such as 2,2-bis((meth)acryloxy phenyl)propane, 2,2-bis(4-(3-(meth)acryloxy-2-hydroxypropoxyphenyl)propane, 2,2-bis(4-(meth)acryloxy ethoxyphenyl)propane, 2,2-bis(4-(meth)acryloxy diethoxyphenyl)propane, 2,2-bis(4-(meth)acryloxy tetraethoxyphenyl)propane, 2,2-bis(4-(meth)acryloxy pentaethoxyphenyl)propane, 2,2-bis(4-(meth)acryloxy polyethoxyphenyl)propane, 2,2-bis(4-(meth)acryloxy dipropooxyphenyl)propane, 2-(4-(meth)acryloxy ethoxyphenyl)-2-(4-(meth)acryloxy phenyl)propane, 2-(4-(meth)acryloxy diethoxyphenyl)-2-(4-(meth)acryloxy triethoxyphenyl)propane, 2-(4-(meth)acryloxy dipropoxyphenyl)-2-(4-(meth)acryloxy triethoxyphenyl)propane and 2,2-bis(4-(meth)acryloxy isopropoxyphenyl)propane to glycyl(meth)acrylate, and a reactant of 1:2 ratio of a bisphenol A or hydrogenated bisphenol A such as a bisphenol A diglycidyl ether(meth)acrylic acid adduct to an epoxy group containing (meth)acrylate.

A specific examples of polyfunctional polymerizable monomers having three or more polymerizable functional groups include tri(meth)acrylates and tetra(meth)acrylates such as trimethylolmethane tri(meth)acrylate, trimethylolethane tri(meth)acrylate, trimethylolpropane tri(meth)acrylate, pentaerythritol tetra(meth)acrylate, phosphazene-based tri(meth)acrylate, isocyanuric acid-based tri(meth)acrylate, pentaerythritol tetra(meth)acrylate, and ditrimethylolpropane tetra(meth)acrylate, urethane-based polymerizable monomer which is derived from a vinyl monomer having a hydroxy group such as glycidol di(meth)acrylate, and a diisocyanate compound such as diisocyanate methylbenzene, 4,4'-diphenylmethane diisocyanate, hexamethylene diisocyanate, trimethyl hexamethylene diisocyanate, diisocyanate methyl cyclohexane, isophorone diisocianate and methyl bis(4-cyclohexylisocyanate), polymerizable monomer having five or more ethylenically unsaturated groups such as dipentaerythritol hydroxypenta(meth)acrylate, polymerizable multifunctional acrylate having polyethylenically unsaturated carbamoylisocianate; urethane bond-containing polymerizable multifunctional acrylate such as phenylglycidyletheracrylate hexamethyllene diisocianate urethane prepolymer, phenylglycidylether toluene diisocyanate urethane prepolymer, pentaerythritol triacrylate toluene diisocyanate urethane prepolymer and pentaerythritol triacrylate isophorone diisocyanate urethane prepolymer; ditrimethylolpropane tetraacrylate, ethoxylated pentaerythritol tetraacrylate, propoxylated pentaerythritol tetraacrylate, pentaerythritol tetra(meth)acrylate, dipentaerythritol tetra(meth)acrylate, dipentaerythritol penta(meth)acrylate, dipentaerythritol hexa(meth)acrylate and trimethyl propane tri(meth)acrylate.

In the present invention, the above described polymerizable monomer and the like can be compounded alone or by mixing two or more types. By mixing two or more polymerizable monomers, it is possible to appropriately adjust the refractive index, the viscosity and various properties of the polymerizable monomer. The compounding amount of the polymerizable monomer may be 1 to 80 % by weight with respect to the dental resin blank for cutting and machining, and may be 10 to 60 % by weight. When the compounding amount of the polymerizable monomer is less than 1 % by weight, it becomes difficult to obtain uniform granular particles to decrease the mechanical strength. Furthermore, when the compounding amount of the polymerizable monomer exceeds 80 % by weight, there is a case where it is not possible to impart sufficient mechanical property or hardness.

In the present invention, it is preferable to compound a polymerization initiator and the like, for the purpose of polymerization and curing. The type of the polymerization initiator to be used is not particular limited, but a thermal polymerization catalyst that generates radical by heating may be used.

As the thermal polymerization catalyst, for example, diacyl peroxides, peroxy esters, dialkyl peroxides, peroxyketals, ketone peroxides and hydroperoxides are effective. Specific examples of the foregoing diacyl peroxides include benzoylperoxide, 2,4-dichlorobenzoylperoxide and m-toluoyl peroxide.

The compounding amount of the polymerization catalyst described above may be 0.01 to 5 parts by weight with respect to 100 parts by weight of the polymerizable monomer, and may be 0.05 to 3 parts by weight. When the compounding amount of the polymerization catalyst is less than 0.01 parts by weight, there is a case where polymerization of the polymerizable monomer is insufficient and it is difficult to impart sufficient mechanical strength. Furthermore, when the polymerization catalyst compounding amount exceeds 5 parts by weight, there is a case where crack or fracture occurs in the molded blank.

In the present invention, uniform polymerization and curing can also be achieved by compounding a chain transfer material into the polymerizable monomer. For the chain transfer material, known compounds can be used without any limitation. Specific examples of the chain transfer agent include: mercaptan compounds such as n-butylmercaptan and n-octylmercaptan; terpenoid-based compounds such as limonene, myrcene, $\alpha$-terpinene, $\beta$-terpinene, $\gamma$-terpinene, terpinolene, $\beta$-pinene and $\alpha$-pinene; and $\alpha$-methylstyrene dimer. Among these chain transfer materials, terpenoid compounds are particularly preferable. Specifically, $\alpha$-terpinene, $\beta$-terpinene and $\gamma$-terpinene are more preferable. The compounding amount of the chain transfer material may be 0.001 to 1 part by weight with respect to 100 parts by weight of the polymerizable monomer, and may be 0.1 to 0.5 parts by weight. When the compounding amount of the chain transfer material is less than 0.001 parts by weight, there is a case where cracks or breaks occur during molding. Furthermore, when the compounding amount of the chain transfer material exceeds 1 part by weight, there is a case where the polymerizable monomer does not polymerize sufficiently.

The viscosity of the binder resin containing the polymerizable monomer and the polymerization catalyst of the present invention is not particularly limited but may be a viscosity capable of impregnating the pores of the porous inorganic filler. The viscosity of the binder resin can be measured using a general rotational viscometer, such as a Type B viscometer. The viscosity of the binder resin may be 0.01 to 1000 Pa·s (at 23 °C), and may be 0.1 to 800 Pa·s (at 23 °C). When the viscosity of the binder resin is less than 0.01 Pa·s, there is a tendency that the mechanical strength of the binder resin decreases, and there is a case where the mechanical property of the dental resin blank for cutting and machining decreases. When the viscosity of the binder resin exceeds 1000 Pa·s, there is a case where the binder resin becomes difficult to impregnate into the pores of the porous inorganic filler to decrease the inorganic filler content and the mechanical property of the dental resin blank for cutting and machining decreases.

The dental resin blank for cutting and machining of the present invention may be compounded with a coloring material in order to impart a shade similar to that of a natural tooth. As the type of the coloring material, any known coloring material can be used without any limitation, and both an inorganic compound-based coloring material and an organic compound-based coloring material may be used.

In the dental resin blank for cutting and machining of the present invention, a known organic filler can be mixed if necessary. Examples of the organic filler include: polymethylmethacrylate (PMMA), polyethylmethacrylate, polypropylmethacrylate, polybutylmethacrylate, polyvinyl acetate, polyethyleneglycol, polypropyleneglycol and polyvinyl alcohol. In addition, examples of an organic composite filler include: a filler prepared by covering the surface of an inorganic filler with a polymerixzable monomer through polymerization, and by subsequently crushing the thus treated inorganic filler so as to have an appropriate particle size, or a filler prepared as particles by beforehand mixing an inorganic filler in a polymerizable monomer, subsequently polymerizing the polymerizable monomer, and successively crushing the thus treated inorganic filler.

In the dental resin blank for cutting and machining of the present invention known various additives can be mixed if necessary. Examples of the additives include polymerization inhibitors, discoloration preventatives, fluorescent agents, UV absorbers, antimicrobial agents and the like, which can be suitably compounded according to purpose.

In the present invention, the specific embodiment used in the step (1) of contacting at least one type of porous inorganic filler with a polymerizable monomer is not particularly limited, and there is no problem as long as a polymerizable monomer can contact with an inorganic filler including a porous inorganic filler.

In the step (1), it is possible to be in a state that the polymerizable monomer and the inorganic filler contact to be kneaded and dispersed. The method for contacting the polymerizable monomer and the inorganic filler is not particularly limited. For example, the inorganic filler may be added onto a pre-measured amount of the polymerizable monomer, or conversely, the polymerizable monomer may be added onto a measured amount of the inorganic filler. There are no particular limitations on the temperature or time during contact. The temperature may be set appropriately to the temperature at which the polymerization catalyst does not react or less. The time is not limited as long as the polymerizable monomer and the inorganic filler disperse without separating. As a kneading method, for example, a method using a magnetic stirrer, a mortar machine, a planetary mixer, a trimix, a twin-screw extruder, a roll pill, a centrifugal mixer or a planetary centrifugal mixer may be used. Particularly, from the viewpoint of facilitating dispersion of the inorganic filler into the polymerizable monomer, an equipment capable of imparting high shear forces may be used. Additionally, dispersants or organic solvents commonly used for dispersion purposes may be added as appropriate. The dispersed state in the present invention means that the inorganic filler exists within the polymerizable monomer as non-agglomerated, non-clumped or non-aggregated particles, not in a clustered or aggregated form. There are no particular limitations on the mixing temperature, but it can be set appropriately to the temperature at which the polymerization catalyst to be used does not react or less. There is also no particular limitations and no problems on the mixing time as long as the polymerizable monomer and the inorganic filler are dispersed without separation. The rotational speed in mixing can be set arbitrarily. The rotational speed can be set, for example, to 5 m/min or higher in terms of peripheral speed based on the rotation speed.

In the present invention, the specific embodiment used in the step (2) of impregnating the porous inorganic filler with the polymerizable monomer to obtain a granular powder is not particularly limited, and it is possible to appropriately set an equipment according to the purpose. For example, after preparing a paste-like product dispersed with the polymerizable monomer and the inorganic filler in the step (1), the polymerizable monomer can be impregnated into the pores of the porous inorganic filler. There are no particular limitations on the temperature or time conditions in the step (2). The temperature can be appropriately set to the temperature at which the polymerization catalyst does not react or less, and there is no problem when the time is at least 1 second or more. As the atmosphere, it may be performed under an air atmosphere, a pressurized atmosphere, or a vacuum atmosphere. From the view point of productivity, a vacuum atmosphere is particularly preferable. The vacuum atmosphere facilitates impregnation of the polymerizable monomer into the pores of the porous inorganic filler.

The degree of vacuum in the vacuum atmosphere may be -750 mmHg to 0 mmHg interms of gauge pressure, preferably -740 to -50 mmHg. When the degree of vacuum is too high, there is a risk that the polymerizable monomer evaporates. It is preferable to set the degree of vacuum appropriately according to the viscosity and component of the polymerizable monomer to be used.

The method used in the step (2) is not particularly limited, but from the viewpoint of productivity, it is preferable to use equipment that allows continuous operation from the step (1).

For example, in the case of using a planetary mixer, the polymerizable monomer may be impregnated into the porous inorganic filler while dispersing the inorganic filler and the polymerizable monomer. There are no particular limitation on the dispersion and/or impregnation conditions, but it is possible to efficiently obtain granular powder by impregnating the polymerizable monomer into the inorganic filler while stirring. The rotational speed in stirring may be set arbitrarily. The rotational speed is preferably 50 m/min or less in terms of peripheral speed based on the rotation speed, and more preferably 30 m/min or less. By setting the rotational speed appropriately, it is possible to obtain uniform particles with the desired particle diameter.

Furthermore, for example, a planetary centrifugal mixer may also be used. The rotational speed in stirring in this case may be set arbitrarily. The rotational speed is preferably 200 m/min or less in terms of peripheral speed based on the rotation speed, and more preferably 150 m/min or less. By appropriately setting the rotational speed, it is possible to achieve a uniform and desired granule size.

The step (1) and the step (2) are preferably performed with temperature adjustment from a view point of productivity. For example, by increasing the temperature to reduce the viscosity of the polymerizable monomer, it is possible to easily impregnate into the pores of the porous inorganic filler. There are no particular limitation on temperature, and it is possible to appropriately set to the temperature at which the polymerization catalyst to be used does not react or less. For example, the temperature in manufacturing can be set between 30 °C and 100 °C. Furthermore, by cooling in extraction of the granular powder, it is possible to improve handling property to enhance productivity. For example, the cooling temperature in manufacturing can be set to 30°C or less.

The granular powder of the present invention means an aggregate in which at least one type of porous inorganic filler is aggregated via a polymerizable monomer. The particle diameter of the granule may be 1 to 5000 $\mu$m, and may be 100 to 2000 $\mu$m. The particle diameter of the granular powder may be measured by analyzing images taken with a general optical microscope or scanning electron microscope. When the particle diameter of the granular powder is less than 1 $\mu$m, there is a case where an internal defect such as pore is more likely to occur in the molded body. Furthermore, when the particle diameter exceeds 5000 $\mu$m, there is a case where workability in placing the granular powder into the mold decreases.

The step (3) of placing the granular powder into a mold and applying pressure and heat to cure of the present invention is not particularly limited as long as it is a method that can mold the desired shape. However, from a viewpoint of productivity, it is preferable to perform by press molding. Examples of the method of press molding include a method that comprises filling the granular powder into a press mold having the desired size and applying pressure by a uniaxial press using upper and lower punches. The press pressure at this time can be appropriately set based on the size of the intended molded body, and the type and the particle diameter of the granular powder. The press pressure may be set to 1 MPa or more in terms of surface pressure, and may be set to 5 MPa or more. When the press pressure is too low, there is a possibility that the granular powder is not densely filled to cause defects within the dental resin blank for cutting and machining.

Examples of another method of press molding in the present invention include cold isostatic pressing (CIP). Generally, in CIP molding, it is possible to apply higher press pressure than uniaxial pressing and to apply pressure uniformly in all three dimensions to the molded body, and therefore it is possible to uniformly mold granular powder.

The heating temperature in the step (3) is not particularly limited as long as it is a temperature that can polymerize and cure the polymerizable monomer and is appropriately set based on the type of used polymerization catalyst.

In the step (3) of the present invention, which includes applying pressure and heat to cure, the applying pressure and the applying heat may be performed simultaneously. Further, it is possible to perform the applying pressure and the applying heat not continuously. For example, after performing press molding to prepare a pre-molded body, the applying heat may be performed to cure. Examples of the method for manufacturing a dental resin blank for cutting and machining via a pre-molded body include a method that comprises placing granular powder into a mold at room temperature, uniaxially press molding to form a pre-molded body, cutting the pre-molded body to the desired mold size and press molding with applying heat to polymerize and cure.

In the present invention, it is possible to layer two or more granular powders having different compositions or shades bay suitably placing into a mold. For example, it is possible to manufacture a dental resin blank for cutting and machining imparted with gradation similar to a natural tooth by preparing granular powders having different shades in advance, layering the granular powders within a mold, and applying pressure to cure.

[Example]

The present invention is described in more detail and specifically with reference to Examples. However, the present invention is not limited to Examples.

[Bending strength test]

Purpose: Evaluation of mechanical strength (bending strength) of dental resin blank for cutting and machining

Method: A plate having a size of $(1.2 \pm 0.2) \times (4.0 \pm 0.2) \times 14$ mm or larger was cut from a prepared dental resin blank for cutting and machining. The surface was polished using #2000 waterproof abrasive paper. These were immersed in distilled water within a sealable container and stored at 37 °C for one day and used as test specimens. The bending strength was measured using an Instron universal testing machine (Instron 5567, manufactured by Instron Corporation) at a distance between supporting points of 12 mm and a crosshead speed of 1 mm/min.

[Evaluation of crack and fracture in molded body]

Purpose: Evaluation of crack and fractur in dental resin blank for cutting and machining

Method: The appearance was visually confirmed for 10 molded dental resin blanks for cutting and machining to evaluate the presence or absence of crack and fracture. The evaluation criteria were as follow.

    A: There were no cracks or fractures.
    B: There were 1 or more cracks or fractures.

[Evaluation of air bubble in molded body]

Purpose: Evaluation of air bubble in dental resin blank for cutting and machining

Method: Using an X-ray transmission device (SMX-31M, Shimadzu Corporation), X-ray radiographs were taken for 10 molded dental resin blanks for cutting and machining. The images were visually confirmed to evaluate the presence or absence of air bubble. The evaluation criteria were as follow.

    A: There were no air bubble having a size of 1 mm or more
    B: There were 1 to 3 air bubbles having a size of 1 mm or more
    C: There were 4 or more air bubbles having a size of 1 mm or more

[Particle circularity]

Particle shape was confirmed from images captured using a scanning electron microscope (JMS-6390LA, JEOL Ltd.). Circularity was calculated by processing the SEM images using an image analysis device. The number of samples to be image processed was 100 or more. In the case of defining the area of the particle obtained by image processing as S and the circumferential length of the particle as L, the circularity was calculated as $(4 \cdot \pi \cdot S)/(L^2)$.

[Porous inorganic filler]

PF-1: porous inorganic filler 1 (shape: spherical, circularity: 0.85, component: zirconium silicate (in terms of zirconium oxide: 15 % by weight), specific surface area: 32 m$^2$/g, pore volume: 0.11 cc/g, 50% particle diameter: 2.5 $\mu$m)

PF-2: porous inorganic filler 2 (shape: spherical, circularity: 0.84, component: zirconium silicate (in terms of zirconium oxide: 18 % by weight), specific surface area: 130 m$^2$/g, pore volume: 0.20 cc/g, 50% particle diameter: 2.9 $\mu$m)

PF-3: porous inorganic filler 3 (shape: spherical, circularity: 0.87, component: zirconium silicate (in terms of zirconium oxide: 17 % by weight), specific surface area: 20 m$^2$/g, pore volume: 0.06 cc/g, 50% particle diameter: 2.4 $\mu$m)

PF-4: porous inorganic filler 4 (shape: spherical, circularity: 0.89, component: zirconium silicate (in terms of zirconium oxide: 1 % by weight), specific surface area: 206 m$^2$/g, pore volume: 0.81 cc/g, 50% particle diameter: 10.5 $\mu$m)

PF-5: porous inorganic filler 5 (shape: spherical, circularity: 0.81, component: strontium silicate (in terms of strontium oxide: 15 % by weight), specific surface area: 35 m$^2$/g, pore volume: 0.12 cc/g, 50% particle diameter: 2.3 $\mu$m)

PF-6: porous inorganic filler 6 (shape: amorphous, circularity: 0.65, component: zirconium silicate (in terms of zirconium oxide: 18 % by weight), specific surface area: 3 m$^2$/g, pore volume: 0.01 cc/g, 50% particle diameter: 3.1 $\mu$m)

PF-7: porous inorganic filler 7 (shape: amorphous, circularity: 0.70, component: zirconium silicate (in terms of zirconium oxide: 16 % by weight), specific surface area: 91 m$^2$/g, pore volume: 0.17 cc/g, 50% particle diameter: 9.5 $\mu$m)

PF-8: porous inorganic filler 8 (shape: spherical, circularity: 0.90, component: zirconium silicate (in terms of zirconium oxide: 16 % by weight), specific surface area: 19 m$^2$/g, pore volume: 0.06 cc/g, 50% particle diameter: 2.2 $\mu$m)

[Other inorganic filler]

NF-1: UF0.4 (shape: amorphous, component: strontium glass, specific surface area: 21 $m^2/g$, 50% particle diameter: 0.4 $\mu$m, manufactured by Schott)
Aerosil R-972 (shape: amorphous (aggregate), component: silica, primary particle diameter: 16 nm, specific surface area: 120 $m^2/g$, manufactured by Aerosil)

[Surface treatment material]

$\gamma$-MPS: $\gamma$-methacryloxypropyl trimethoxysilane (manufactured by Shin-Etsu Chemical Co., Ltd.)

[Polymerizable monomer]

UDMA: 2,2,4-trimethylhexamethylene bis (2-carbamoyloxyethyl) dimethacrylate
BP-2EM: EO adduct dimethacrylate of bisphenol A
TEGDMA: triethylene glycol dimethacrylate

[Polymerization catalyst]

BPO: benzoyl peroxide
DMBH: 2,5-dimethyl-2,5-di (t-butylperoxy) hexane

[Chain transfer material]

$\alpha$-terpinene: p-menta-1,3-diene (manufactured by Tokyo Chemical Industry Co., Ltd.)
$\gamma$-terpinene: p-menta-1,4-diene (manufactured by Tokyo Chemical Industry Co., Ltd.)

By using the above polymerizable monomers, polymerization catalyst and chain transfer material, a binder resin was prepared.

[Preparation of binder resin 1]

UDMA: 70 parts by weight, TEGDMA: 30 parts by weight, BPO: 0.5 parts by weight and $\alpha$-terpinene: 0.1 parts by weight were mixed to prepare (B1) binder resin. The viscosity of the (b1) binder resin was 0.3 Pa·s (23 °C).

[Preparation of binder resin 2]

BP-2EM: 100 parts by weight, DMBH: 0.5 parts by weight and $\gamma$-terpinene: 0.3 parts by weight were mixed to prepare (B2) binder resin. The viscosity of the (b2) binder resin was 1.4 Pa·s (23 °C).

[Preparation of binder resin 3]

BP-2EM: 100 parts by weight, DMBH: 5 parts by weight and $\alpha$-terpinene: 1 part by weight were mixed to prepare (B3) binder resin. The viscosity of the (b3) binder resin was 1.2 Pa·s (23 °C).

[Preparation of binder resin 4]

UDMA: 100 parts by weight and DMBH: 0.5 parts by weight were mixed to prepare (B4) binder resin. The viscosity of the (b4) binder resin was 10.8 Pa·s (23 °C).

[Example 1]

The (PF-1) porous inorganic filler (100 parts by weight) was surface-treated with γ-MPS (6 parts by weight). The surface-treated filler: 78 parts by weight and the (B1) binder resin: 22 parts by weight were kneaded and dispersed using a double planetary mixer. Subsequently, the mixing vessel was stirred (rotation speed: 5 m/min) under vacuum condition (vacuum level: -700 mm Hg) until a granular powder (particle diameter: 572 $\mu$m) was obtained. The obtained granular powder was filled into a mold (14.5 mm × 14.5 mm × 18.0 mm) and uniaxially press-molded (surface pressure: 50 MPa, temperature: 130 °C) to polymerize and cure to prepare a dental resin blank for cutting and machining.

[Example 2]

The (PF-2) porous inorganic filler (100 parts by weight) was surface-treated with γ-MPS (20 parts by weight). The surface-treated filler: 70 parts by weight, Aerogel R-972 (5 parts by weight) and the (B1) binder resin: 25 parts by weight were kneaded and dispersed using a double planetary mixer. Subsequently, the mixing vessel was stirred (rotation speed: 10 m/min) under vacuum condition (vacuum level: -650 mm Hg) until a granular powder (particle diameter: 236 $\mu$m) was obtained. Thereafter, a dental resin blank for cutting and machining was prepared in the same manner as in Example 1.

[Example 3]

The (PF-3) porous inorganic filler (100 parts by weight) was surface-treated with γ-MPS (6 parts by weight). The surface-treated filler: 80 parts by weight and the (B1) binder resin: 20 parts by weight were kneaded and dispersed using a double planetary mixer. Subsequently, the mixing vessel was stirred (rotation speed: 10 m/min) under vacuum condition (vacuum level: -730 mm Hg) until a granular powder (particle diameter: 452 $\mu$m) was obtained. Thereafter, a dental resin blank for cutting and machining was prepared in the same manner as in Example 1.

[Example 4]

The (PF-4) porous inorganic filler (100 parts by weight) was surface-treated with γ-MPS (25 parts by weight). The surface-treated filler: 60 parts by weight, NF-1 (8 parts by weight) and the (B1) binder resin: 32 parts by weight were kneaded and dispersed using a double planetary mixer. Subsequently, the mixing vessel was stirred (rotation speed: 10 m/min) under vacuum condition (vacuum level: -600 mm Hg) until a granular powder (particle diameter: 942 $\mu$m) was obtained. Thereafter, a dental resin blank for cutting and machining was prepared in the same manner as in Example 1.

[Example 5]

The (PF-4) porous inorganic filler (100 parts by weight) was surface-treated with γ-MPS (25 parts by weight). The surface-treated filler: 8 parts by weight, NF-1 (71 parts by weight) and the (B1) binder resin: 21 parts by weight were kneaded and dispersed using a double planetary mixer. Subsequently, the mixing vessel was stirred under vacuum condition (vacuum level: -600 mm Hg) until a granular powder (particle diameter: 628 $\mu$m) was obtained. The obtained granular powder was filled into a mold (14.5 mm × 14.5 mm × 18.0 mm) and uniaxially press-molded (surface pressure: 1 MPa, temperature: 150 °C) to polymerize and cure to prepare a dental resin blank for cutting and machining.

[Example 6]

The (PF-1) porous inorganic filler (100 parts by weight) was surface-treated with γ-MPS (6 parts by weight). The surface-treated filler: 55 parts by weight, NF-1 (23 parts by weight) and the (B2) binder resin: 22 parts by weight were kneaded and dispersed using a double planetary mixer. Subsequently, the mixing vessel was stirred (rotation speed: 9 m/min) under vacuum condition (vacuum level: -700 mm Hg) until a granular powder (particle diameter: 292 $\mu$m) was obtained. The obtained granular powder was filled into a mold (14.5 mm × 14.5 mm × 18.0 mm) and uniaxially press-molded (surface pressure: 50 MPa, temperature: 150 °C) to polymerize and cure to prepare a dental resin blank for cutting and machining.

[Example 7]

The (PF-2) porous inorganic filler (100 parts by weight) was surface-treated with γ-MPS (20 parts by weight). The surface-treated filler: 75 parts by weight and the (B2) binder resin: 25 parts by weight were kneaded and dispersed using a double planetary mixer. Subsequently, the mixing vessel was stirred (rotation speed: 12 m/min) under atmospheric condition until a granular powder (particle diameter: 1492 $\mu$m) was obtained. Thereafter, a dental resin blank for cutting and machining was prepared in the same manner as in Example 6.

[Example 8]

The (PF-1) porous inorganic filler (100 parts by weight) was surface-treated with γ-MPS (6 parts by weight). The surface-treated filler: 55 parts by weight, NF-1 (23 parts by weight) and the (B2) binder resin: 22 parts by weight were kneaded and dispersed using a double planetary mixer. Subsequently, the mixing vessel was stirred (rotation speed: 5 m/min) under vacuum condition (vacuum level: -700 mm Hg) until a granular powder (particle diameter: 292 μm) was obtained. Thereafter, a dental resin blank for cutting and machining was prepared in the same manner as in Example 6.

[Example 9]

The (PF-2) porous inorganic filler (100 parts by weight) was surface-treated with γ-MPS (20 parts by weight). The surface-treated filler: 75 parts by weight and the (B2) binder resin: 25 parts by weight were stirred (rotation speed: 188 m/min) in a mixing vessel under atmospheric condition using a rotating-revolving mixer (Awatori neritaro) until a granular powder (particle diameter: 855 μm) was obtained. Thereafter, a dental resin blank for cutting and machining was prepared in the same manner as in Example 6.

[Example 10]

The (PF-5) porous inorganic filler (100 parts by weight) was surface-treated with γ-MPS (10 parts by weight). The surface-treated filler: 75 parts by weight and the (B2) binder resin: 25 parts by weight were kneaded and dispersed using a double planetary mixer. Subsequently, the mixing vessel was stirred (rotation speed: 10 m/min) under vacuum condition (vacuum level: -700 mm Hg) until a granular powder (particle diameter: 650 μm) was obtained. The obtained granular powder was filled into a mold (14.5 mm × 14.5 mm × 18.0 mm) and uniaxially press-molded (surface pressure: 10 MPa, temperature: 150 °C) to polymerize and cure to prepare a dental resin blank for cutting and machining.

[Example 11]

The (PF-6) porous inorganic filler (100 parts by weight) was surface-treated with γ-MPS (1 part by weight). The surface-treated filler: 90 parts by weight and the (B4) binder resin: 10 parts by weight were kneaded and dispersed using a double planetary mixer. Subsequently, the mixing vessel was stirred (rotation speed: 10 m/min) under vacuum condition (vacuum level: -700 mm Hg) until a granular powder (particle diameter: 650 μm) was obtained. Thereafter, a dental resin blank for cutting and machining was prepared in the same manner as in Example 6.

[Example 12]

The (PF-7) porous inorganic filler (100 parts by weight) was surface-treated with γ-MPS (20 parts by weight). The surface-treated filler: 65 parts by weight and the (B1) binder resin: 35 parts by weight were kneaded and dispersed using a double planetary mixer. Subsequently, the mixing vessel was stirred (rotation speed: 3 m/min) under vacuum condition (vacuum level: -700 mm Hg) until a granular powder (particle diameter: 2100 μm) was obtained. Thereafter, a dental resin blank for cutting and machining was prepared in the same manner as in Example 1.

[Example 13]

The (PF-8) porous inorganic filler (100 parts by weight) was surface-treated with γ-MPS (6 parts by weight). The surface-treated filler: 75 parts by weight and the (B3) binder resin: 25 parts by weight were kneaded and dispersed using a double planetary mixer. Subsequently, the mixing vessel was stirred (rotation speed: 30 m/min) under vacuum condition (vacuum level: -700 mm Hg) until a granular powder (particle diameter: 90 μm) was obtained. Thereafter, a dental resin blank for cutting and machining was prepared in the same manner as in Example 6.

[Comparative Example 1]

Other inorganic filler (NF-1) (100 parts by weight) was surface-treated with γ-MPS (6 parts by weight). The surface-treated filler: 75 parts by weight and the (B1) binder resin: 25 parts by weight were kneaded and dispersed using a double planetary mixer to prepare a paste-like product. Subsequently, the prepared paste-like product was filled into a mold (14.5 mm × 14.5 mm × 18.0 mm) and uniaxially press-molded (surface pressure: 50 MPa, temperature: 100 °C) to polymerize and cure to prepare a dental resin blank for cutting and machining.

[Comparative Example 2]

The (PF-4) porous inorganic filler (100 parts by weight) was surface-treated with γ-MPS (25 parts by weight). The surface-treated filler: 60 parts by weight, NF-1 (8 parts by weight) and the (B1) binder resin: 32 parts by weight were kneaded and dispersed using a double planetary mixer to prepare a paste-like product. Subsequently, the prepared paste-like product was filled into a mold (14.5 mm × 14.5 mm × 18.0 mm) and uniaxially press-molded (surface pressure: 50 MPa, temperature: 100 °C) to polymerize and cure to prepare a dental resin blank for cutting and machining.

[Table 1]

|  | Bending strength (Mpa) | Crack and Fracture | Bubble |
|---|---|---|---|
| Example 1 | 218 | A | A |
| Example 2 | 200 | A | A |
| Example 3 | 206 | A | A |
| Example 4 | 180 | A | A |
| Example 5 | 196 | A | A |
| Example 6 | 227 | A | A |
| Example 7 | 209 | A | A |
| Example 8 | 222 | A | A |
| Example 9 | 192 | A | A |
| Example 10 | 207 | A | A |
| Example 11 | 185 | A | A |
| Example 12 | 173 | A | A |
| Example 13 | 216 | A | A |
| Comparative Example 1 | 166 | B | C |
| Comparative Example 2 | 117 | A | C |

The above results shows that, in the dental resin blank for cutting and machining of the present invention, there are no cracks and fractures, and no air bubbles and high mechanical strength is imparted. Therefore, it is considered that the manufacturing method of a dental resin blank for cutting and machining of the present invention is a manufacturing method of the dental resin blank for cutting and machining which has excellent versatility and productivity and is imparted with high mechanical strength.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this disclosure without difficulty. Accordingly, any such changes and modifications are intended to be included in the scope of the embodiments.

[Industrial applicability]

According to the present invention, it is possible to provide a dental resin blank for cutting and machining which has excellent versatility and productivity and is imparted with high mechanical strength.

**Claims**

1. A manufacturing method of a dental resin blank for cutting and machining, comprising

    step (1) of contacting at least one type of porous inorganic filler with a polymerizable monomer,
    step (2) of impregnating the porous inorganic filler with the polymerizable monomer to obtain a granular powder, and
    step (3) of placing the granular powder into a mold and applying pressure and heat to cure.

2. The manufacturing method of a dental resin blank for cutting and machining according to claim 1, wherein a specific surface area of the porous inorganic filler is 10 to 300 $m^2/g$.

3. The manufacturing method of a dental resin blank for cutting and machining according to claim 1, wherein a pore volume of the porous inorganic filler is 0.01 to 0.20 cc/g.

4. The manufacturing method of a dental resin blank for cutting and machining according to claim 2, wherein a pore volume of the porous inorganic filler is 0.01 to 0.20 cc/g.

5. The manufacturing method of a dental resin blank for cutting and machining according to any one of claims 1 to 4, wherein
the step (2) of impregnating the porous inorganic filler with the polymerizable monomer to obtain a granular powder is performed under vacuum condition.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H10323353 A
- WO 2014021343 A
- JP 2023035918 A